# EUROPEAN PATENT APPLICATION

(11) **EP 1 329 505 A1**
(43) Date of publication of application: **23.07.2003**
(21) Application number: 01980933.4
(22) Date of filing: 29.10.2001
(51) Int. Cl.: C12N 15/09, C12Q 1/68

(54) **METHOD OF GENE AMPLIFICATION**

(30) Priority: 27.10.2000 JP 2000369279
(71) Applicant: Mishima, Kenji, Fukuoka-shi, Fukuoka 811-0121 (JP); Supercritical Co., Ltd., Fukuoka-shi, Fukuoka 812-0051 (JP)
(72) Inventor: MISHIMA, Kenji, Fukuoka-shi, Fukuoka 811-0121 (JP)
(74) Representative: Koepe, Gerd L., Dipl.-Chem.
(86) International application number: JP0109490
(87) International publication number: WO02034905

(57) **Abstract**

A method of gene amplification which comprises annealing primers to a target gene of an animal, a plant, etc. and then amplifying the gene by using a heat-resistant nucleic acid polymerase such as Taq polymerase or Pfu polymerase, characterized by using at least one high-pressure fluid selected from the group consisting of supercritical fluids such as carbon dioxide, subcritical fluids, high-pressure gases and liquids as the reaction medium. It is preferable to amplify the gene by using a surfactant-coated enzyme obtained by coating a heat-resistant nucleic acid polymerase with a surfactant and controlling the reaction temperature.

SEQUENCE LISTING

<110> MISHIMA Kenji

<120> Method for gene amplification

<130> P2-01M08199

<160> 2

<210> 1

<211> 22

<212> DNA

<213> Artificial Sequence

<400> 1

gccagggttt tcccagtcac ga                                                22

<210> 2

<211> 22

<212> DNA

<213> Artificial Sequence

<400> 2

gagcggataa caatttcaca gg                                                22

## Description

### Technical Field

The present invention relates to methods of gene amplification using nucleic acid polymerase, and more specifically relates to gene amplification methods that use at least one high-pressure fluid selected from the group consisting of supercritical fluids, subcritical fluids, high-pressure gases, and liquids as the reaction medium.

### Background Art

In recent years, the correlation between the genetic sequences unique to living organisms and illness has gained attention. Decoding the sequence of nucleic acids such as deoxyribonucleic acid (DNA) and ribonucleic acid (RNA) is at the core of the field of genetic research. To decode genetic information such as DNA or RNA, it is necessary to amplify the DNA or RNA to a concentration that allows analysis (R.W. Old and S. B. Primrose, "Principle of Gene Manipulation," Blackwell Scientific Publication (1989)).

One effective approach to gene amplification is to perform amplification using appropriate heat-resistant enzymes while controlling the reaction temperature. In particular, the advances in genetic manipulation technologies in recent years have been coupled with numerous reports on methods related to the amplification of DNA and RNA (Tanba Mineo, "Chemical Synthesis of DNA," Hirokawa Shoten (1992)).

Polymerase Chain Reaction (PCR), one of the more common methods, is a method for directly amplifying short fragments of RNA or DNA from a genome. The gene amplification by PCR is performed in the presence of a target gene, primers (probes) complementary to the target gene, nucleic acids, and heat-resistant nucleic acid polymerases. In the amplification, the process of (i) dissociating the target gene, (ii) annealing (hybridizing) the primers (or probes) to the target gene, and (iii) performing a chain-elongation reaction (gene synthesis reaction) is performed as one cycle, and this cycle is repeated so as to amplify the gene. The dissociation and chain elongation of the gene are kept at appropriate temperatures. However, problems with PCR are that it takes time from the amplification to the detection of the gene, the special enzymes that are employed make PCR expensive, and the amplification efficiency and specificity may change due to the design of the primer complementary to the target gene.

Also, single-stranded DNA macromolecules are generally known to have complex structures. There are the problems that it takes time to hybridize a macromolecule of a single-stranded DNA probe having such a complex structure to the target DNA, and that the heat-resistant enzymes used in amplification have poor correcting activity, resulting in a high frequency of errors, for example.

One method to solve these problems is a gene amplification method (Japanese Laid-Open Patent Publication (Tokkai) No. 2000-201687) for forming a double-stranded macromolecule using a plurality of pairs of probes that are complementary to one another at three or more sites and carrying out hybridization so that these probes cross one another in alternation. However, even with this method, although the target gene can be amplified and detected using antigen-antibody reactions more efficiently than in conventional methods, there were the problems that the speed was too slow and the procedure was too arduous for practical purposes.

On the other hand, one method for synthesizing bio-materials that has gained attention is a method for synthesizing bio-materials using as the reaction medium a supercritical fluid or a high-pressure gas, which are not normally used in methods for synthesizing bio-materials. Carbon dioxide is particularly advantageous because it is generally innocuous, it has a critical temperature of 304.2 K, which is relatively close to room temperature, it allows the reaction to be controlled simply by manipulating the pressure, and there is not the problem of residual solvents, for example. For these reasons, extensive research regarding enzyme reactions and reactions without catalyst using supercritical carbon dioxide has been carried out and widely reported.

For example, reports have been made on radical polymerization reactions of acrylic esters having fluoroalkyl groups (DeSimone et al., Science, 265, 356 (1994)), hydrogenation reactions without catalyst (Jessop et al., Nature, 368, 231 (1994)), and enzyme reactions (O. Aaltonen and M. Rantakyla, CHEMTECH, 4, 240 (1991)) in supercritical carbon dioxide. The present inventors have also already developed techniques for synthesizing and cleaving genes using supercritical carbon dioxide (Japanese Laid-Open Patent Publication (Tokkai) No. H11-206371).

However, there has yet to be a report on gene amplification using a supercritical fluid such as carbon dioxide or a high-pressure gas, for example, and using heat-resistant nucleic acid polymerases while controlling the temperature.

### Disclosure of Invention

In light of the foregoing, it is an object of the present invention to provide a technique with which a supercritical fluid is used to amplify genes and with which errors at the time of gene amplification are reduced.

The present invention relates to a method of gene amplification for annealing primers to a target gene and amplifying the gene using a heat-resistant nucleic acid polymerase, wherein at least one high-pressure fluid selected from the group consisting of supercritical fluids, subcritical fluids, high-pressure gases, and liquids serves as a reaction medium.

In a preferable embodiment, the fluid is a supercritical fluid, and by controlling at least one of working pressure and temperature, the gene is amplified and errors in the synthesized gene are reduced.

In another preferable embodiment, the fluid is a supercritical fluid of carbon dioxide.

It is further preferable that the heat-resistant nucleic acid polymerase is coated with a surfactant or a lipid, and even more preferable that the surfactant is isooctylsodium sulfasuccinate, sorbitan monostearate, or polyoxyethylene octylphenylether, and the lipid is lecithin.

### Brief Description of Drawings

Fig. 1 is a diagram showing a device for carrying out gene amplification using supercritical carbon dioxide as the reaction medium.
Fig. 2 is an agarose gel electrophoresis photograph of DNA fragments amplified by Taq polymerase using the device shown in Fig. 1. For comparison, the size markers M and fragments P amplified by a general method are shown.
Fig. 3 is an agarose gel electrophoresis photograph of DNA fragments amplified by the device of Fig. 1 using (A) KOD polymerase and (B) Pfu polymerase.
Fig. 4 is an electrophoresis photograph comparing an amplified product (P) obtained through a conventional PCR technique and a PCR amplified product (S) obtained using supercritical carbon dioxide.

### Best Mode for Carrying Out the Invention

In light of the foregoing, it is an object of the present invention to provide a technology with which high-pressure fluids are used to amplify genes and with which errors during gene amplification are reduced.

The reaction medium in the present invention is characterized in that it is a high-pressure fluid. A fluid used herein means a liquid or a gas in a fluid state. High-pressure is used to mean a pressure higher than atmospheric pressure, and normally means at least 7.3 MPa. The temperature is below the temperature at which the heat-resistant nucleic acid polymerases are inactivated, and generally is not more than 100 °C. However, this depends on the enzyme, for example, KOD polymerase, is not inactivated even when the temperature exceeds 100 °C.

Examples of the fluid include supercritical fluids, subcritical fluids, high-pressure gases, and liquids. The fluid may be composed of a single gas or liquid, a mixture of two or more different types of gases or different liquids, or a mixture of gases and liquids.

Although there are no particular limitations regarding the supercritical fluids, subcritical fluids, high-pressure gases, and liquids that are used in the present invention, it is preferable that a compound (gas) that is known to be useful as a supercritical fluid, such as carbon dioxide, methane, ethane, propane, ethylene, and ammonia, is used. These fluids not only have the operational advantage that they do not inactivate the gene during the reaction, but they also can be easily recovered, making them further advantageous in that they do not pollute the environment.

A preferable fluid is a supercritical fluid, of which the supercritical fluid carbon dioxide is most preferably used because of its excellent advantages: it has a supercritical temperature of 304.2 K, which is relatively near room temperature, it is not harmful, it allows the reaction to be controlled simply by manipulating the pressure, and it lacks the problems of residual solvents, for example.

During the gene amplification reaction, an organic solvent such as the following may be added to the fluid: an aliphatic alcohol such as methanol, ethanol, and 1-propanol; an aliphatic ketone such as acetone, methylethylketone, and cyclohexanone; and an aromatic hydrocarbon such as benzene, toluene, xylene, tetrahydrofuran, and dimethyl formamide. These are only illustrative examples, and are added to an extent that the effects of the present invention are not diminished.

In the present invention, "target gene" is used to refer to a gene that serves as the template in the gene amplification reaction, and as long as it is DNA or RNA, there are no particular limitations as to its origin or size, although it is preferably DNA of a size up to approximately 10 kb.

There are also no particular limitations regarding the primers (probes) used in the present invention, and normally a pair of primers, one near the 5' terminal and one near the 3' terminal of the target gene (template), are used. The primers can be synthesized to correspond to the target gene, and if possible, commercially available primers may also be used. For example, if pBluescript SK+ is used as the target gene, then primers that can be used include the KS primer, M13 (lacZ) primer, SK primer, T3 primer, and T7 primer.

There are no particular limitations to the heat-resistant nucleic acid polymerase used in the present invention, and generally used heat-resistant nucleic acid polymerases may be adopted. For example, examples include Pfu polymerase (derived from Pyrococcus furiosus), Taq polymerase (derived from Themus aquaticus), Tth polymerase (derived from Themus thermophilius HBB), and KOD polymerase (derived from Pyrococcus Kodakaraensis KOD1), although there are no limitations to these.

Although these heat-resistant nucleic acid polymerases can be employed as they are, it is preferable that they are coated with a surfactant or lipids in order to keep them active even in high-pressure supercritical fluid and to efficiently perform the reaction. Examples of a surfactant that can be used as a coating include isooctylsodium sulfasuccinate, sorbitan monostearate, and polyoxyethylene octylphenylether, although there are no limitations to these. An example of a lipid that can be used as a coating is lecithin.

The coating of the heat-resistant nucleic acid polymerase is achieved by mixing a 2 µg/ml enzyme aqueous solution and a solvent (preferably isooctane) in which a surfactant (preferably isooctylsodium sulfasuccinate) is dissolved so that the weight ratio of enzyme aqueous solution:surfactant:solvent is 10:1:25, mixing for ten minutes with a homogenizer, and then drying the mixed solution at room temperature.

The gene amplification reaction is carried out by preparing the target gene, primers (probes), a mixture of nucleic acids (dNTP: mixture of dATP, dCTP, dTTP, and dGTP), and a heat-resistant nucleic acid polymerase that is preferably coated with a surfactant or a lipid, in a reaction cell, and for example, introducing carbon dioxide, and preferably carrying out the reaction in a supercritical state. The reaction is controlled by adjusting at least one of the working pressure and the temperature.

The target gene is denatured and annealed at approximately 90 to 98 °C and preferably 93 to 95 °C, which is achieved by adjusting the pressure or the temperature.

The amplification reaction (chain elongation reaction) is performed at approximately 65 to 85 °C and preferably at 69 to 76 °C, which is achieved by adjusting the pressure or the temperature.

Thus, gene amplification can be carried out efficiently by controlling the temperature in each of the steps. Also, along with the increase in working pressure, there is a tendency for a decrease in amplification errors.

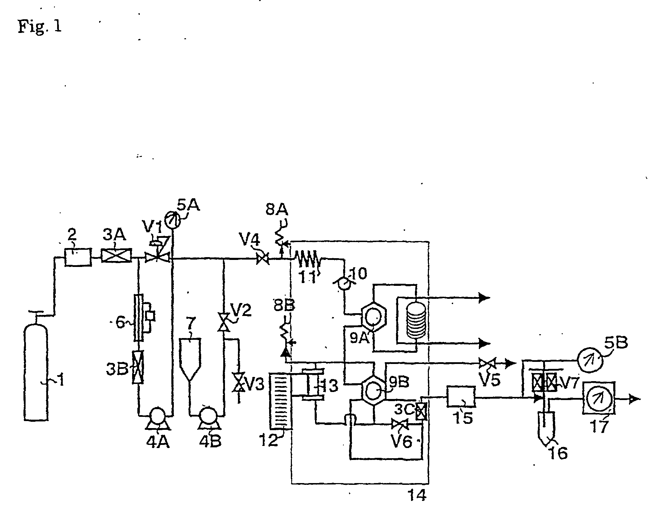

A high-pressure reaction device is used in the present invention because the reaction is performed in a high-pressure fluid. An example of an amplification device that can be used in the present invention is a device that uses supercritical fluid, such as the one shown in Fig. 1. Hereinafter, the amplification device is described with reference to Fig. 1. The amplification device includes a pressurizing portion, which is constituted by the components from a cylinder 1 to a stop valve V2, an amplification reaction portion, which is constituted by the components from a stop valve V4 to a stop valve V6, and an analysis and collection portion located downstream of the amplification reaction portion.

The pressurizing portion has two pressurizing pumps 4A and 4B for carbon dioxide as the supercritical fluid, for example, and for the solution. The carbon dioxide cylinder 1 is a siphon-type cylinder for delivering liquid carbon dioxide to the pressurizing pump 4A. A dry pipe 2 is arranged between the cylinder 1 and the pump 4A in order to remove the water in the liquid carbon dioxide that is delivered from the cylinder 1. The specifications of the dry pipe 2 are: material SUS316, maximum allowable working pressure 20 MPa, inner diameter 35.5 mm, length 310 mm. Also, for the drying agent it is possible to use a molecular sieve (1/16 inch pellet) made by GL Sciences K.K., for example. The liquid carbon dioxide from which the water has been removed by the dry pipe 2 is cooled by ethylene glycol, which is maintained at approximately -5 °C by a cooling unit 6 (BL-22, made by Yamato Scientific Co., Ltd.), and delivered to the pressurizing pump 4A (gas supply pump).

For the pressurizing pump 4A it is possible to use the high-pressure single-plunger pump APS-5L made by GL Sciences K.K. (maximum pressure 58.8 MPa, normal pressure 49.0 MPa, flow amount 0.5 to 5.2 ml · min⁻¹). A cooling unit for preventing vaporization of the liquid carbon dioxide is fitted to the head portion of the pump for delivering carbon dioxide. Additives, deblocking agents, or condensing agents are filled into a reagent vessel 7, which is disposed upstream of the pressurizing pump 4B. The eluent that is pressurized by the pressurizing pump 4B passes through the stop valve V2 and is mixed with the pressurized carbon dioxide before the stop valve V4 and is then supplied to the column separation portion. A stop valve V3 is arranged for discharging the eluent. The bonnet integrated-type flow adjustment stop valve SS-OKS2BKB made by Whitey is used for the stop valve. Also, the FT4-10 model by GL Sciences K.K. is used as a filter 3A for preventing contamination by impurities, such as dirt, between the dry pipe 2 and the cooling unit 6. The average pore diameter of the filter is approximately 10 µm.

The pressure inside the system is set to any pressure by a pressure adjusting valve V1. For the pressure adjusting valve V1, 26-1722-24 made by TESCOM is used. This valve can control the pressure within the system to ±0.1 MPa, and its maximum allowable working pressure is 41.5 MPa (415 bar). The pressure within the system is measured using a Bourdon-type pressure gauge 5A (LCG-350 made by GL Sciences K.K.; maximum working pressure 34.3 MPa). A high limit contact output terminal is attached to this pressure gauge, and is set so as to cut the power of the pressurizing pump 4A at a designated pressure. Also, the pressure gauge is tested using the Economy Pressure Gauge PE-33-A (strain gauge type, precision ±0.3%) made by Shisokken K.K.

The stop valve V4 is arranged between the pressurizing portion and the extraction portion in order to control the pressure of the pressurizing portion. The 2 Way Valve 02-0120 (maximum working pressure 98.0 MPa) made by GL Sciences K.K. is used as the stop valve. Also, a safety valve 8A is arranged between the pressurizing portion and the column separation portion in order to ensure safety. The safety valve is a spring-type valve made by NuPro, and is adjusted and tested so that it operates when the pressure inside the system is 34.3 MPa. It should be noted that 1/16 inch stainless steel pipes (material SUS316, outer diameter 1.588 mm, inner diameter 0.8 mm) are used for the piping of the pressurizing portion except for the area from the cylinder 1 to the filter 3A, and 1/8 inch stainless steel pipes (material SUS316, outer diameter 3.175 mm, inner diameter 2.17 mm) are used for all other portions.

The liquid carbon dioxide and the eluent supplied from the pressurizing portion are delivered to a pre-heating column (not shown) that is provided in a heating plate 11. The pre-heating column is for heating the solvent (carbon dioxide or the like) to the equilibrium temperature so as to turn it into a supercritical fluid, and is a 1/8 inch stainless steel pipe (material SUS316, outer diameter 3.175 mm, inner diameter 2.17 mm, length approximately 4 m) that has been modified to a spiral shape with a diameter of 55 mm and a length of 140 mm and arranged inside an air bath 14. The carbon dioxide that has been turned into a supercritical fluid in the pre-heating column and the reaction solution are passed through a stopper 10 (SS-CHS4-10 made by NuPro: maximum working pressure 41.2 MPa) for preventing backflow of fluid, and are introduced into a reaction column 13. It should be noted that the reaction column 13 is arranged in a hexagonal valve 9A, and has been set so that the pipe can be cleaned by switching the hexagonal valve 9A, except during the amplification operation in the reaction column 13. Moreover, an injector is internally provided in a hexagonal valve 9B (Rheodyne 7125 made by GL Sciences K.K.), and this injector allows reagents to be introduced. Also, stop valves V5 and V6 are arranged in order to discharge the pressure inside the column. It should be noted that the high-pressure resistance switch valve HPV-6 made by GL Sciences K.K. (maximum working pressure 34.3 MPa) is used as the hexagonal valve 9A.

The bonnet integrated-type flow adjustment stop valve SS-OKS2BKB made by Whitey is also used for the stop valves V5 and V6. After passing through the column, the pressure is measured by 5B and is adjusted to the setting pressure by a flow adjustment valve V7. The pressure gauge is a Bourdon-type gauge, and for it LCG-350 (maximum working pressure 34.3 MPa) made by GL Sciences K.K. is used. The pressure gauge is tested using the Economy Pressure Gauge PE-33-A (strain gauge type, precision ±0.3% FS, FS: kgf · cm⁻²) made by Shisokken K.K. Also, a safety valve 8B is arranged on the upstream side of the reaction column 13 for the purpose of preventing explosions due to rising pressure within the column. The spring-type valve 177-R3AKI-G made by NuPro is used for the safety valve 8A, which is adjusted and tested so that it activates when the pressure inside the system is 34.3 MPa. The supercritical fluid (carbon dioxide) in which the reagent is dissolved is discharged outside the system using the flow adjustment valve V7.

For the analysis and collection portion, a back pressure regulator made by JASCO Corporation (880-81 model; automatic pressure adjustment valve, working pressure range 0 to 49.0 MPa, pressure adjustment precision ±2%) is used for the flow adjustment valve V7. With this valve, the flow of the supercritical fluid is adjusted so as to reduce the pressure. A high-pressure filter 3B is arranged on the upstream side of the flow adjustment valve so as to prevent blockage within the pipe due to condensation of the reagent. 2TF-7 made by NuPro (average pore diameter 7 µm) is used as the filter 3B. Also, a heater is arranged in the flow adjustment valve V7 to prevent the condensation of the reagent as the pressure is reduced and the formation of dry ice due to the supercritical fluid (carbon dioxide). Furthermore, by making the outlet of the pipe an oscillatory-type outlet, it is possible to prevent blockage of the pipe due to precipitate. After the pressure is reduced by the flow adjustment valve V7, the sample that has been precipitated is recovered by a trap 16. Also, the flow is measured by a flow meter 17. For the flow meter, the integrating wet gas meter W-NK-0.5B (measurement precision 0.1 ml) made by Shinagawa Corporation is used. Amplified compounds can also be confirmed using an ultraviolet light detector 15 (UV-970 made by JASCO Corporation).

The air bath 14 is an airtight system that includes the reaction column 13, and is provided with the heating plate 11 as a heating (temperature increasing) device and a cooling plate 12 as a cooling (temperature lowering) device. The air bath 14 is also provided with a Peltier element that has a temperature control function and a temperature sensor. The temperature of the air bath 14 can be controlled to ±0.1 K of the measurement temperature by the temperature controller DB1000 made by Chino Corporation, for example. The temperature is measured using the platinum resistance thermometer 1TPF483 made by Chino Corporation. A Peltier element is used as the cooling device. The cooling medium that is supplied to the Peltier element is always cooled to 4 °C, for example, and is stored in a cooling container. Thus, the plurality of temperature controls that are required for the amplification reaction are possible.

With this device, in the reaction column 13 of the air bath 14, the annealing (hybridization) is generally carried out under the conditions of approximately 8 to 20 MPa and approximately 90 to 98 °C, and the gene amplification reaction is carried out under the conditions of approximately 8 to 15 MPa and approximately 65 to 85 °C.

### Working Examples

Hereinafter, working examples of the present invention are provided to describe the invention in greater detail, however, the present invention is not limited to these working examples.

### First Working Example

Using the device of Fig. 1, the gene amplification reaction was performed. 50 ng of pBluescript SK+ (made by Toyobo Co., Ltd.) to be amplifies, 30 pmol each of the primer Forward M13 (lacZ) Primer 5'-GCCAGGGTTTTCCCAGTCACGA-3' (Sequence I.D. No.1) and the primer Reverse M13 (lacZ) Primer 5'-GAGCGGATAACAATTTCACAGG-3' (Sequence I.D. No.2), 0.2 mM of dNTP (mixture), and 2.5 U of the heat-resistant nucleic acid polymerase, Taq polymerase, coated with the surfactant isooctylsodium sulfasuccinate (created under the above-mentioned conditions) were put into the reaction cell in the reactoin column in the air bath.

The stop valve V4 was opened to introduce carbon dioxide into the cell, and the pressure inside the cell was set to 10 MPa. At this time, the upper limit pressure was adjusted by the flow adjustment valve V7. Also, the temperature was adjusted so that the temperature controller for the carbon dioxide was set to 35 °C and the flow adjustment valve V7 was set to 80 °C. The cooling medium supplied to the Peltier element was 4 °C.

Annealing was performed at 94 °C for 0.5 minutes and the chain elongation reaction was performed at 74 °C for 0.5 minutes. To cool the temperature from 94 °C to 74 °C, the heater was turned off so as to allow cooling by the cooling plate. On the other hand, to raise the temperature from 74 °C to 94 °C, the heater was turned on so as to apply heat by the heating plate.

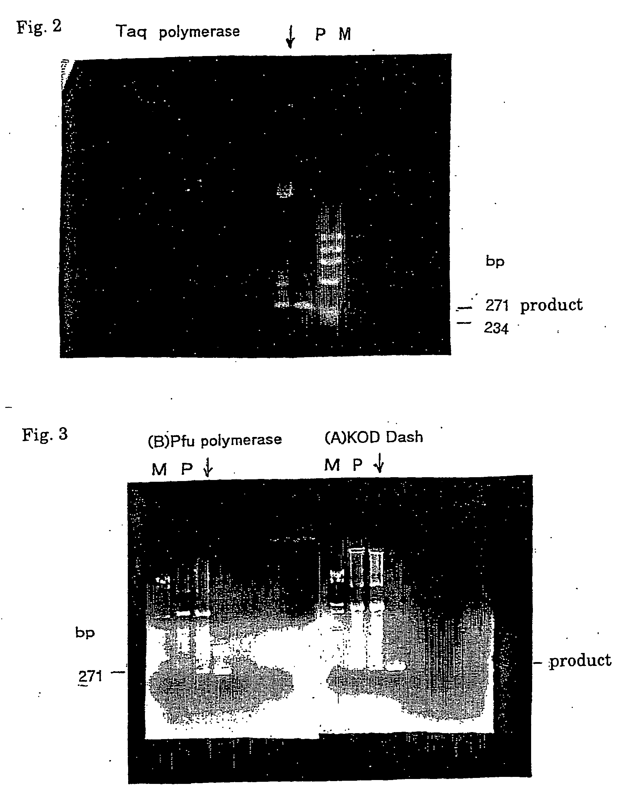

After repeating the cycle of heating and cooling at least 25 times, the amplified gene fragments were recovered from the flow adjustment valve V7. The recovered gene was subjected to agarose gel electrophoresis (Fig. 2: ). The results of the electrophoresis are shown in Fig. 2. From Fig. 2, a band can be confirmed on the same line as the product of 271 bp (Fig. 2: P) that was amplified by PCR under atmospheric pressure with pBluescript SK+ serving as the template, indicating that the gene fragment of pBluescript SK+ was amplified in supercritical carbon dioxide. In Fig. 2, M is the molecular weight marker.

### Second Working Example

In the same manner as in the first working example, pBluescript SK+ served as the template and amplification was performed using KOD dash polymerase and Pfu polymerase. Fig. 3 shows an electrophoresis diagram (Fig. 3: ) in which amplification was performed with (A) KOD dash polymerase and (B) Pfu polymerase. In both instances, it is clear that the gene fragment of pBluescript SK+ was amplified.

### Third Working Example

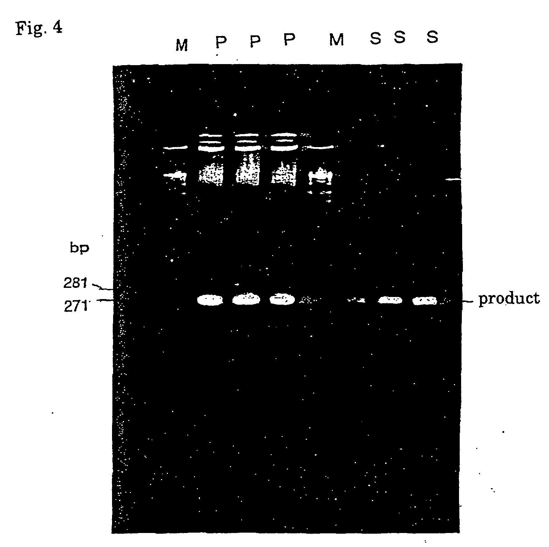

In the same way as the first working example and using KOD dash polymerase as the enzyme, gene amplification was carried out with pBluescript SK+ as the template. For comparison, a conventional PCR reaction performed in atmospheric pressure was carried out under the same temperature conditions and reaction time using KOD dash polymerase. The results are shown in Fig. 4. It is shown that compared to the amplified product in aqueous solution (Fig. 4: P), the amplified product in supercritical carbon dioxide according to the present invention (Fig. 4: S) presents bands with a narrower distribution. This result indicates that amplification errors are decreased when genes are amplified in supercritical carbon dioxide, resulting in products that appear as sharp bands.

### Industrial Applicability

According to the present research, by using at least one type of high-pressure fluid selected from the group consisting of supercritical fluids, subcritical fluids, high-pressure gases, and liquids serving as the reaction medium, it is possible to amplify genes by controlling the temperature while annealing primers to a target gene of, for example, a virus, microorganism, animal, or plant and using a surfactant-coated enzyme that is obtained by coating a heat-resistant nucleic acid polymerase such as Taq polymerase or Pfy polymerase with a surfactant. Also, the amount of organic solvent can be reduced, and gene amplification can be controlled by manipulating the working pressure and the temperature.

The method of gene amplification of the present invention allows genes to be efficiently amplified and for example allows a gene to be detected through an antigen-antibody reaction if it has antigenicity, and thus can find application in genetics-related manufacturing and in the medical and pharmaceutical fields.

## Claims

1. A method of gene amplification comprising annealing primers to a target gene and amplifying the gene using a heat-resistant nucleic acid polymerase, wherein at least one high-pressure fluid selected from the group consisting of supercritical fluids, subcritical fluids, high-pressure gases, and liquids serves as a reaction medium.

2. The method of gene amplification according to claim 1, wherein the fluid is a supercritical fluid, and by controlling at least one of working pressure and temperature, the gene is amplified and errors in the amplified gene are reduced.

3. The method according to claim 1 or 2, wherein the fluid is a supercritical fluid of carbon dioxide.

4. The method according to any one of claims 1 to 3, wherein the heat-resistant nucleic acid polymerase is coated with a surfactant or a lipid.

5. The method according to claim 4, wherein the surfactant is isooctylsodium sulfasuccinate, sorbitan monostearate, or polyoxyethylene octylphenylether, and the lipid is lecithin.
